(19) **Europäisches Patentamt / European Patent Office / Office européen des brevets**

(11) **EP 1 561 384 B1**

(12) **EUROPEAN PATENT SPECIFICATION**

(45) Date of publication and mention of the grant of the patent:
**10.10.2007  Bulletin 2007/41**

(21) Application number: **03758846.4**

(22) Date of filing: **23.10.2003**

(51) Int Cl.:
**A23J 3/16** (2006.01)  **A23J 1/00** (2006.01)

(86) International application number:
**PCT/JP2003/013585**

(87) International publication number:
**WO 2004/043160 (27.05.2004 Gazette 2004/22)**

(54) **FRACTIONATED SOYBEAN PROTEIN AND PROCESS FOR PRODUCING THE SAME**

FRAKTIONIERTES SOJABOHNENPROTEIN UND HERSTELLUNGSVERFAHREN DAFÜR

PROTEINE DE SOJA FRACTIONNE ET PROCEDE DE PRODUCTION DE CELLE-CI

(84) Designated Contracting States:
**AT BE BG CH CY CZ DE DK EE ES FI FR GB GR HU IE IT LI LU MC NL PT RO SE SI SK TR**

(30) Priority: **12.11.2002  JP 2002328243**

(43) Date of publication of application:
**10.08.2005  Bulletin 2005/32**

(73) Proprietor: **Fuji Oil Company, Ltd.**
**Osaka-shi,**
**Osaka 542-0086 (JP)**

(72) Inventors:
• **ISHIKAWA, Masahiro,**
**Fuji Oil Company, Limited**
**Izumisano-shi,**
**Osaka 598-8540 (JP)**

• **HIROTSUKA, Motohiko,**
**Fuji Oil Company, Limited**
**Izumisano-shi,**
**Osaka 598-8540 (JP)**

(74) Representative: **Hayes, Adrian Chetwynd et al**
**Boult Wade Tennant,**
**Verulam Gardens**
**70 Gray's Inn Road**
**London WC1X 8BT (GB)**

(56) References cited:
EP-A- 1 535 517          WO-A-03/063608
WO-A1-02/28198          JP-A- 5 043 597
JP-A- 55 124 457          JP-A- 2002 114 694
US-A- 4 368 151          US-A- 4 370 267

**Description**

The present invention relates to a process for producing a 7S globulin-rich fraction and an 11S globulin-rich fraction from a solution containing soybean protein.

Background Art

Soybean storage protein is precipitated at about pH 4.5 and can be relatively easily separated from components other than the protein. This is referred to as a soybean protein isolate and, in many cases, soybean protein in this form is utilized in the food industry. The soybean storage protein is further divided into 2S, 7S, 11S and 15S globulins according to sedimentation constants in ultracentrifugation analysis. Among them, 7S globulin and 11S globulin are predominant constituent protein components of the globulin fractions (note: 7S globulin and 11S globulin are classification names in a sedimentation method and substantially correspond to β-conglycinin and glycinin according to immunological nomenclature, respectively), and both of them have specific different properties such as viscosity, coagulability, surface activity, etc. Then, fractionation of soybean protein into 7S globulin-rich fraction and 11S globulin-rich fraction makes it possible to utilize properties of respective protein components, and it is expected to expand industrial utilization of proteins.

7S Globulin and 11S globulin are composed of several subunits. 7S Globulin is composed of three subunits, i.e., α, α' and β subunits. 11S Globulin is composed of a pair of an acidic polypeptide (A) and a basic polypeptide (B) each of which has several subunits. The composition ratio of these subunits in most common conventional soybeans is typically about 1 : 2 in the ratio of 7S globulin to 11S globulin as determined from the densitometry area ratio of migration patterns obtained by SDS-polyacrylamide gel electrophoresis. The properties of 7S globulin and 11S globulin resemble in their molecular weights and charge states. In particular, both globulins are diversified due to combinations of subunits, and properties thereof range to some extent to thereby overlap each other. Accordingly, it is not easy to efficiently separate these globulins with little contamination to one another.

WO 03/063608 discloses a process for the extraction, purification and enzymatic modification of soy 7S globulin alpha' subunit for use as hypocholesterolemizing agent.

US 4,368,151 discloses a method for 7S and 11S vegetable protein fractionation and isolation. This method involves precipitating the 11S protein at a pH 5.8-6.3 in the presence of carefully controlled concentrations of water-soluble salts and sulfurous ions. The enriched 7S whey may then be adjusted to a pH 5.3-5.8 to precipitate substantially all of the remaining water-soluble 11S protein from the whey and an enriched 7S fraction may then be recovered from the whey.

EP 1 323 353 A1 discloses a method for fractionating a soybean protein into highly pure 7S globulin and 11S, the method comprising warming a solution containing a soybean protein under a weakly acidic condition followed by fractionating at pH5.6 to 6.6 into a soluble fraction and an insoluble fraction.

Conventionally known fractionation methods are as follows. That is, these methods include a method utilizing a difference in isoelectric points: only 7S globulin is extracted in the vicinity of the isoelectric point of 11S globulin (JP 55-124457 A); a method utilizing a difference in reactivity with a calcium salt: a 7S globulin-rich fraction is extracted by addition of a small amount of calcium during extraction (JP-A 48-56843 A); a method utilizing a difference in solubility at a certain pH and ionic strength: 7S globulin is prepared by removing insoluble fractions in the presence of sodium chloride or potassium chloride in a pH region of 1.2 to 4.0 (JP 49-31843 A), 7S globulin and 11S globulin are separated by adjusting pH of a slurry after isoelectric point precipitation to 5.0 to 5.6 while adjusting sodium chloride concentration to 0.01 to 0.2 M (JP 58-36345 A), and 7S globulin is fractionated by adjusting the ionic strength of a protein-containing solution to 0.2 to 0.3 and the pH to 4.8 to 5.2 to remove insoluble fractions, and then adjusting the ionic strength to less than 0.2 and the pH to 4.6 to 5.0 (JP 5-43597 A); and a method utilizing a cold-precipitation phenomenon and a reducing agent, etc.: this utilizes a phenomenon that the solubility of 11S globulin is lowered at a low temperature (referred to as cryo-precipitation phenomenon), and a soybean protein raw material is treated in the presence of a sulfurous acid compound, glutathione compound or cysteine compound in an aqueous system at pH 6.5 or higher, followed by adjusting pH to 5.5 to 7.0 and a temperature to 22°C or lower to fractionate into a 7S globulin-rich soluble fraction and an 11S globulin-rich insoluble fraction (JP 61-187755 A).

These known fractionation methods skillfully utilize a difference in solubility between 7S globulin and 11S globulin due to pH, ionic strength, the presence of a certain salt, temperature, etc. However, there are such problems that these known fractionation methods are unsuitable for an industrially applicable fractionation method because, for example, separation by a high centrifugal force is required for clear fractionation. Thus, problems still remain in practice. For example, in the method of JP 61-187755 A, cryo-precipitation phenomenon highly depends on a temperature and it is necessary to cool a reaction mixture to about 5°C, which results in such a practical problem that a large amount of a sulfurous acid compound, etc. should be added to separate fractions with an industrially available low centrifugal force, as well as which results in such a problem of fractionation precision that a little amount of 11S globulin is contaminated in a soluble fraction.

For the purpose of obtaining 7S globulin-rich protein, isolation of protein from 11S globulin-defect soybeans, i.e., a 7S globulin-rich seeds produced by breeding has been studied (Breeding Science, 46, 11, 1996), and its utility (Breeding Science, 50, 101, 2000) and patent (US 6,171,640 B1) can also be found.

As described above, a method for separating a 7S globulin-rich fraction and a 11S globulin-rich fraction by which an mutual contamination between soluble and insoluble fractions is reduced and by which a production in an industrial scale is accomplished conveniently and efficiently has been researched and developed.

On the other hand, it has been reported by Samoto et al. that, in soybean-derived protein, there is a protein component having high affinities with polar lipid as a constituent of a cytoplasmic membrane as well as a protein body or oil body membrane (oil-body-associated protein) which amounts to as high as about 35 % of an industrially produced soybean protein isolate (Biosci. Biotechnol. Biochem., 62 (5), 935-940 (1998)). The oil-body-associated protein is a general term of protein components consisting mainly of membrane protein, especially those whose molecular weights measured by SDS-polyacrylamide gel electrophoresis are mainly 34 kDa, 24 kDa and 18 kDa, and contains about 10 to 12% by weight of polar lipid which are extractable with a 2 : 1 polar solvent mixture of chloroform : ethanol.

Conventional fractionation is focusing only on 7S and 11S globulins, and in many cases, pays no attention to the oil-body-associated protein which is contaminating each fraction, since the oil-body-associated protein can not be identified as positively as 7S and the 11S globulins when analyzed by SDS-polyacrylamide gel electrophoresis and is underestimated frequently. In other words, a purity when determined only by SDS polyacrylamide gel electrophoresis becomes higher frequently than the actual purity, and the behavior of the oil-body-associated protein should be taken into account for the purpose of obtaining 7S or 11S globulin at a really high purity. Thus, a conventional fractionation into the two fractions 7S globulin/11S globulin handles a purity of a fraction only as a ratio between 7S globulin and 11S globulin. However, each fraction is associated with the oil-body-associated protein, and in many cases its actual state is a relatively crude fraction having a slightly lower purity whose protein composition is characterized by a large amount of the oil-body-associated protein.

While the present inventors have proposed a method which makes industrial fractionation of 7S globulin and 11S globulin possible only by heat-treating under acidic conditions (WO 02/28198 A1), as a result of an intensive study, it has been found that pH for separating a soluble fraction containing 7S globulin from an insoluble fraction containing 11S globulin can be lowered by combining heat treatment of a solution containing soybean protein under acidic conditions with adjustment of an ionic strength, thereby further facilitating separation of the soluble fraction and the insoluble fraction.

The present invention proposes a novel fractionation method between 7S globulin and 11S globulin, in particular, one object of the present invention is a highly accurate and efficient fractionation method which can be performed in an industrial scale. Another object of the present invention is to provide protein fractions having characteristic features with less contamination of the oil-body-associated protein and having high purities of 7S globulin and 11S globulin.

Disclosure of the Invention

The present invention relates to:

(1) A process for producing soybean protein, which is comprises heating a solution containing the soybean protein under acidic conditions, and then fractionating it into a soluble fraction and an insoluble fraction at an ionic strength of 0.02 or more and pH of 4.5 or higher but lower than 5.6;
(2) The process according to (1), wherein the solution containing the soybean protein is an aqueous slurry of defatted soybeans, defatted soybean milk obtained from the slurry, a slurry of acid-precipitated soybean protein, or a solution of soybean protein isolate;
(3) The process according to (1), wherein the acidic conditions are those at pH 3.8 to 6.8;
(4) The process according to (1), wherein the heating is performed at 30 to 75°C;
(5) The process according to (1), which further comprises fractionating 7S globulin protein from the soluble fraction obtained by the fractionation in (1), wherein a ratio of 7S globulin/(11S globulin + 7S globulin) of said 7S globulin protein is 0.5 or more, and a content of a polar lipid extracted by a mixed solvent of chloroform and methanol (chloroform : methanol = 2 : 1) in the solid content of said 7S globulin protein is 1% by weight or less;
(6) The process according to (1), which further comprises fractionating 11S globulin protein from the insoluble fraction obtained by the fractionation in (1), wherein a ratio of 11S globulin/(11S globulin + 7S globulin) of said 11S globulin protein is 0.7 or more, and a content of a polar lipid extracted with a mixed solvent of chloroform and methanol (chloroform : methanol = 2 : 1) in the solid content of said 11S globulin protein is 2% by weight or less.

Best Mode for Performing the Invention

The method of the present invention is characterized in that, in comparison with the method which makes industrial fractionation of 7S globulin and 11S globulin possible only by heat-treating under acidic conditions (WO 02/28198 A1), pH for separating a soluble fraction containing 7S globulin from an insoluble fraction containing 11S globulin can be lowered by combining heat treatment of a solution containing soybean protein under acidic conditions with adjustment of an ionic strength, thereby further facilitating separation of the soluble fraction and the insoluble fraction. The above acidic conditions are preferably those at pH 3.8 to 6.8, the heating temperature is preferably 30 to 75°C, the ionic strength is preferably 0.02 or more, and the separation of the soluble fraction from the insoluble fraction is preferably performed at a pH of 4.5 or more but lower than 5.6. Whereby a 7S globulin-rich soluble fraction containing less oil-body-associated protein can be obtained, and further, an 11S globulin and oil-body-associated protein-rich insoluble fraction can be obtained. It is possible to selectively dissolve and separate 11S globulin with keeping the oil-body-associated protein insoluble by applying weak shear stress to the above insoluble fraction in an aqueous solution at an approximately neutral pH (pH 6.5 to 7.5), thereby dissolving or extracting 11S globulin in the insoluble fraction to obtain a 11S globulin-rich fraction containing less oil-body-associated protein.

Further, in the present invention, a protein fraction with a low phytic acid content of 1.2% or less of phytic acid per protein can be obtained by decomposing phytic acid by a phytase during the production step. The separation efficiency can be further improved by performing the phytase treatment before separation of the soluble fraction and the insoluble fraction.

The soluble fraction obtained by the above production process has a ratio of 7S globulin/(7S globulin + 11S globulin) of 0.5 or more, and a high purified 7S globulin fraction with the above ratio of 0.8 or more, 0.85 or more, or 0.9 or more can be readily obtained by appropriately selecting pH for separating the soluble fraction and the insoluble fraction. Another fraction, i.e., the insoluble fraction, has a 11S globulin/(11S globulin + 7S globulin) ratio of 0.7 or more, and a high purified 11S globulin fraction with the above ratio of 0.8 or more, 0.85 or more, or 0.90 or more can be readily obtained by appropriately selecting the pH for separating the soluble fraction and the insoluble fraction.

Herein, the ratio of 7S globulin/(11S globulin + 7S globulin) or 11S globulin/(11S globulin + 7S globulin) can be determined from the area ratio between the corresponding fractions by measuring the migration pattern obtained by SDS-polyacrylamide electrophoresis with a densitometry.

The oil-body-associated protein is present in a proportion of about 30 to 35% in acid-precipitated protein, when the protein is obtained precisely using sodium sulfate from the acid-precipitated protein that is substantially free from denaturation (Biosci. Biotechnol. Biochem., 62(5), 935-940 (1988)). In view of the fact that polar lipid extracted by a mixed solvent of chloroform and methanol in a ratio of 2 : 1 (volume ratio) is present in a proportion of 3 to 4% by weight in the solid content of the acid-precipitated protein and in a proportion of 10 to 12% by weight in the oil-body-associated protein, the above polar lipid (hereinafter, sometimes, abbreviated as "chloroform-methanol oil content") is unevenly distributed in the oil-body-associated protein of the acid-precipitated protein, and the amount of the oil-body-associated protein can be calculated to be 10 fold by weight of the chloroform-methanol oil content. However, this calculation is applicable to a subject material prepared via a defatting step with hexane, etc., and in case of a subject material which is not subjected to hexane extraction, the calculation is firstly applicable after defatting with hexane.

The 7S globulin-rich soluble fraction obtained in the present invention contains 1% or less of the chloroform-methanol oil content, and the content of the oil-body-associated protein is estimated to be 10% or less. The 11S globulin-rich fraction extracted from the insoluble fraction contains 2% or less of the chloroform-methanol oil content, and the content of the oil-body-associated protein is calculated to be 20% or less.

The assay method used in the present invention will be described below.

* Crude protein: The nitrogen content was determined by Kjeldahl method, and it was multiplied by a coefficient of 6.25 to convert it into the amount of crude protein.

* Separation-precipitation rate of insoluble fraction: A separation characteristic analyzer LUMiFuge 114 manufactured by L.U.M. Co. was used. A sample solution (0.5 ml) containing the insoluble fraction was placed in a polystyrene rectangular cell, the cell was centrifuged at 100G, and the moving rate of the interface with a transmittance of 20% was defined as the separation-precipitation rate.

* SDS-polyacrylamide electrophoresis: A gradient gel with a gel concentration of 10% to 20% according to the method by Laemmli (Nature, 227, 680 (1970)) was used to analyze protein. The applied amount of the protein was 6 $\mu$g in terms of a solid content, and the gel was stained with Coomassie Brilliant Blue R-250.

* Phytic acid: The amount of phytic acid was assayed according to Alii Mohamed method (Cereal Chemistry 63, 475-478 (1986)).

* Chloroform-methanol oil content: About 50 fold in volume of a mixed solution of chloroform-methanol (volume ratio, 2 : 1) was added to a dried sample, and the weight ratio of the solid content extracted by refluxing the solvent was measured as the chloroform-methanol oil content.

* Purity (SPE standard): The migration pattern obtained by the above SDS-polyacrylamide electrophoresis was measured with a densitometry. The area ratio of the desired fraction relative to the total area of the pattern was defined to be the purity (SPE standard). The content of 7S globulin refers to the total content of $\alpha$, $\alpha$' and $\beta$ subunits, and the content of 11S globulin refers to the total content of the acidic polypeptide (A) and basic polypeptide (B).

* Corrected purity: The corrected purity was calculated from the above-obtained purity (SPE standard) as follows by taking the oil-body-associated protein contaminated therein into consideration. That is, since a sample contains the oil-body-associated protein corresponding to 10 fold by weight of the chloroform-methanol oil content in addition to 7S globulin and 11S globulin, assuming A (%) represents the purity (SPE standard), a corrected purity is calculated relative to the total protein including 7S globulin, 11S globulin and the oil-body-associated protein:

```
Corrected purity (%) = (100 (%) - chloroform-methanol

oil content (%) × 10) × A (%) / 100
```

* Ionic strength: Conductivity of a solution was measured using a conductivity meter (2%/°C, with a temperature conversion function), and a molar concentration of NaCl corresponding to the measured conductivity was defined to be the ionic strength.

Preferred embodiments of the present invention will be described below.

As raw material soybeans used in the present invention, any soybeans of commercially available soybeans, or soybeans deficient in a specified fraction produced by bleeding or genetic engineering can be used. While the solution containing soybean protein may be any of an aqueous slurry of defatted soybeans (hereinafter referred to as a defatted soybean slurry), defatted soybean milk obtained from the slurry, a slurry of acid-precipitated soybean protein (hereinafter referred to as a curd slurry), or a solution of a soybean protein isolate, in particular, to use an aqueous slurry of defatted soybeans is preferable since the soluble fraction and the insoluble fraction are readily separated. A solution of non-denatured or scarcely denatured soybean protein is preferable in order to fractionate into a 7S globulin-rich fraction and an 11S globulin-rich fraction.

The heating of the solution containing soybean protein under acidic conditions is performed at pH 3.8 to 6.8, more preferably pH 4.0 to 6.6, further preferably pH 4.2 to 6.2 at a temperature of 30 to 75°C, more preferably 35 to 65°C, and further preferably 40 to 60°C. The ionic strength of 0.02 or more facilitates separation of the soluble fraction and the insoluble fraction, and the higher ionic strength can further facilitate separation of the soluble fraction and the insoluble fraction. However, since an ionic strength should be adjusted to less than 0.2 for isoelectric point precipitation of 7S globulin from the soluble fraction after separation when the ionic strength of 0.2 or more is used for separating the fraction, an ionic strength of 0.02 or more but less than 0.2 is recommended for avoiding complex separation procedures. The 7S globulin-rich soluble fraction and the 11S globulin-rich insoluble fraction can be obtained when the soluble fraction is separated from the insoluble fraction at pH of 4.5 or more but less than 5.6. Separation of the 7S globulin-rich fraction and the 11S globulin-rich fraction can be further facilitated by decomposing phytic acid contained in the soybean protein with phytase during the production process, particularly in any steps before the soluble fraction and the insoluble fraction are separated. The phytase treatment is simplified and facilitated when it is performed simultaneously with the heat treatment under acidic conditions. The optimum conditions for the phytase treatment are usually at pH of 3.5 to 9.0, and a temperature of 20 to 70°C for 5 minutes to 3 hours with about 0.1 to 100 units of a phytase per protein weight (g), though the conditions somewhat may vary depending on the origin of the phytase. The phytase activity of 1 unit is defined by the amount of the enzyme required for releasing 1 $\mu$mole of phosphoric acid from phytic acid as the substrate in 1 minute at the initial stage of the enzymatic reaction at pH 5.5 and 37°C.

Unlike to the pH and the temperature in the heat treatment described above, the length of the duration of the heating has no serious effect on the difficulty experienced in a separation process, and thus is of a less significance. A pH departing out of the range from pH 3.8 to 6.8 or a temperature out of the range from 30°C to 75°C in the heating treatment may lead to a difficulty in separating into 7S globulin and 11S globulin.

After the heating treatment, the fractionation may be performed at the same temperature, although it is preferable to ensure a cooling for controlling any microbes. The fractionation may be accomplished by using a known separation procedure (such as filtration and centrifugation), and an easy separation can also be accomplished especially by using a continuous centrifuge (such as a decanter). It is a matter of course that the use of a non-continuous centrifuge such as a batch centrifuge is not excluded.

The accuracy of fractionation into the 7S globulin-rich soluble fraction and the 11S globulin-rich insoluble fraction according to the present invention can be evaluated based on the pattern obtained by SDS-polyacrylamide electrophoresis (the purity is in SPE standard).

However, since the purity based on SPE standard may be underestimated due to low stainability of the oil-body-associated protein on the SDS-polyacrylamide electrophoresis, the corrected purity obtained by the above equation:

EP 1 561 384 B1

$$\text{Corrected purity (\%) = (100 (\%) - chloroform-methanol}$$

$$\text{oil content (\%)} \times 10) \times A (\%) / 100$$

is considered to be closer to the true purity.

After separation, the soluble fraction can be used as the 7S globulin-rich fraction as such or after concentration, neutralization, sterilization or drying. The concentration can be performed, for example, by adjusting the ionic strength at less than 0.2 with pH adjustment of 4.0 to 5.0 to separate and recover the resulting insoluble fraction. Usually, the soluble fraction is further diluted with water, neutralized, sterilized by heating, and dried. The heat sterilization can be performed by a known method such as HTST, UHT treatment. Of course, the fraction may be treated with an enzyme such as a protease in a solution state according to a particular purpose.

11S Globulin can be dissolved or extracted from the insoluble fraction after separation using an appropriately neutral aqueous solution (at about pH 6.5 to 7.5) in order to separate 11S globulin from the insoluble oil-body-associated protein [including "okara (insoluble residue from soybean milk or tofu production)" component when the insoluble fraction contains it]. At this time, it is preferable to dissolve or extract 11S globulin by applying shear stress as week as possible in order to selectively dissolve or extract 11S globulin while preventing soulblization of the oil-body-associated protein fraction. A high-G centrifuge at centrifugal force of about 4,000 G or more, preferably about 5,000 G or more, is favorably used for separating extracted or dissolved 11S globulin from the oil-body-associated protein, thereby making it possible to obtain a protein fraction with a chloroform-methanol oil content of 2% or less in the solid content while maintaining the proportion of 11S globulin of 0.7 or more relative to the total amount of 11S globulin and 7S globulin.

The 11S globulin-rich fraction, which is dissolved or extracted using an approximately neutral aqueous solution and contains 2% or less of the extracted chloroform-methanol oil content, can be used as the 11S globulin-rich fraction as such or after concentration, neutralization, sterilization or drying. The concentration can be performed, for example, by adjusting the soluble fraction at pH 4.5 or more but less than 5.6 to separate and recover the resulting insoluble fraction. The insoluble fraction is usually further diluted with water, neutralized and sterilized by heating followed by drying. The heat sterilization is usually performed by a known method such as HTST and UHT treatment. Of course, the insoluble fraction may be treated with an enzyme such as a protease in a solution state according to a particular purpose.

The following Examples will further illustrate the present invention.

Example 1

To scarcely denatured and defatted soybeans (1 part by weight, nitrogen solubilization index (NSI): 91), which was obtained by flaking soybeans and extracting their oil with an extraction solvent, n-hexane, to separate and remove the oil, was added extraction water (10 parts by weight, ion-exchange water). The mixture was stirred with a homo-mixer at 22°C and extracted for 40 minutes with maintaining at pH 7.2 by addition of a 20% sodium hydroxide solution. Then, the extract was filtered by a filter cloth, and centrifuged at 5,000 G for 10 minutes to remove insolubles, thereby obtaining defatted-soybean milk. The soybean milk was adjusted to pH 4.5 with 35% hydrochloric acid, and centrifuged at 3,000 G for 10 minutes to obtain acid-precipitated protein. Ion-exchange water was added to the acid-precipitated protein so that the protein content was 5% as dry weight, and the solution was homogenized with Polytron (manufactured by KINEMATICA AG)(hereinafter referred to as a curd slurry). The ionic strength of the curd slurry was adjusted to 0.14 with sodium chloride, and stirred at 22°C for 30 minutes, and the pH of the curd slurry was adjusted to 5.3 with a 20% aqueous sodium hydroxide solution, followed by stirring at 22°C for 20 minutes. Then, the curd slurry was heated to 50°C and, after stirring at 50°C for 10 minutes, the curd slurry was immediately cooled to 22°C. The time required from the start of heating to cooling to 22°C was 25 minutes. After further stirring at 22°C for 15 minutes, the separation-precipitation rate of the insoluble fraction was measured using LUMiFuge 114. At the same time, the soluble fraction and insoluble fraction obtained by centrifugation at 5,000 G for 5 minutes were assayed by SDS-polyacrylamide electrophoresis, and the ratios of 7S globulin and 11S globulin (the fraction area ratio obtained by measuring migration patterns of SDS-polyacrylamide electrophoresis with a densitometer) were determined.

The separation-precipitation rate of the insoluble fraction is shown in Table 1, and the ratios of 7S globulin and 11S globulin in the soluble fraction and insoluble fraction are shown in Table 2.

Comparative Example 1

The ionic strength of a 5% curd slurry prepared according to the same manner as in Example 1 was adjusted to 0.14 with sodium chloride. The curd slurry was stirred at 22°C for 30 minutes, adjusted to pH 5.3 with a 20% aqueous sodium hydroxide solution, and then stirred at 22°C for 60 minutes. According to the same manner as in Example 1, the separation-precipitation rate of the insoluble fraction was measured, and the soluble fraction and insoluble fraction obtained by centrifugation at 5,000 G for 10 minutes were assayed by SDS-polyacrylamide electrophoresis to determine the ratios of 7S globulin and 11S globulin.

The separation-precipitation rate of the insoluble fraction is shown in Table 1, and the ratios of 7S globulin and 11S globulin in the soluble fraction and insoluble fraction are shown in Table 2.

Table 1

Separation-precipitation rate of insoluble fraction

|  | With heating | Without heating |
| --- | --- | --- |
| Separation-precipitation rate (μm/sec) | 146 | 57 |

Table 2

Ratio of 7S globulin and 11S globulin (7S:11S)

|  | Soluble fraction | Insoluble fraction |
| --- | --- | --- |
| With heating | 98:2 | 15:85 |
| Without heating | 98:2 | 13:87 |

As seen from the results in Example 1 and Comparative Example 1, it is clear that separation of the soluble fraction containing highly purified 7S globulin and the insoluble fraction containing highly purified 11S globulin can be further facilitated by heating the curd slurry under acidic conditions.

Example 2

The ionic strength of a 5% curd slurry prepared according to the same manner as in Example 1 was adjusted to 0.14 with sodium chloride. The curd slurry was stirred at 22°C for 30 minutes without adjusting pH (about pH 4.5), and heated to 50°C, followed by immediately cooling to 22°C. The time required from the start of heating to cooling to 22°C was 15 minutes. After cooling, pH of the curd slurry was adjusted to 5.5 with a 20% aqueous sodium hydroxide solution followed by stirring for 15 minutes. The separation-precipitation rate of the insoluble fraction was measured according to the same manner as in Example 1, and the ratios of 7S globulin and 11S globulin in the soluble fraction and insoluble fraction were determined.

The ratios of 7S globulin and 11S globulin in the soluble fraction and insoluble fraction are shown in Table 3, and the separation-precipitation rate of the insoluble fraction is shown in Table 4.

Comparative Example 2

A 5% curd slurry prepared according to the same manner as in Example 1 was subjected to heat treatment according to the same manner as in Example 2 (pH was not adjusted; the slurry was stirred at 22°C for 30 minutes, and heated to 50°C followed by immediate cooling to 22°C) without adjusting the ionic strength (ionic strength: 0.013). Then, pH of the curd slurry was adjusted to 5.9 with a 20% aqueous sodium hydroxide solution (a fraction having a similar ratio of 7S globulin and 11S globulin was not able to be obtained unless pH for separating the soluble fraction and the insoluble fraction was raised when the ionic strength was low). After stirring for 15 minutes, the separation-precipitation rate of the insoluble fraction was measured according to the same manner as in Example 1, and the ratios of 7S globulin and 11S globulin in the soluble fraction and insoluble fraction were measured.

The ratios of 7S globulin and 11S globulin in the soluble fraction and insoluble fraction are shown in Table 3, and the separation-precipitation rate of the insoluble fraction is shown in Table 4.

Table 3

The ratio of 7S globulin and 11S globulin (7S : 11S)

| Ionic strength | Separation pH | Soluble fraction | Insoluble fraction |
| --- | --- | --- | --- |
| 0.14 | 5.5 | 84:16 | 18:82 |
| 0.013 | 5.9 | 82:18 | 15:85 |

Table 4

Separation-precipitation ratio of insoluble fraction

| Ionic strength | 0.14 | 0.013 |
|---|---|---|
| Separation-precipitation rate ($\mu$m/sec) | 204 | 48 |

As seen from the results in Example 2 and Comparative Example 2, it is clear that separation of the soluble fraction containing 7S globulin from the insoluble fraction containing 11S globulin can be further facilitated by raising the ionic strength and lowering the separation pH.

As seen from the results in the above Examples and Comparative Examples, it is clear that separation of the soluble fraction containing 7S globulin and the insoluble fraction containing 11s globulin is further facilitated by combining heat treatment of a soybean protein containing solution under acidic conditions with adjustment of the ionic strength, as compared with the heat treatment only under acidic conditions or adjustment of the ionic strength only.

Example 3

Extraction water (ion-exchange water, 10 parts by weight, 22°C) was added to 1 part by weight of scarcely denatured and defatted soybeans defatted according to the same manner as in Example 1 (hereinafter referred to as a defatted soybean slurry). The ionic strength was adjusted to 0.17 with sodium chloride, and the curd slurry was stirred with a propeller at 22°C for 30 minutes without adjusting the pH. Then, pH was adjusted to 5.3 with 35% hydrochloric acid, and the crude slurry was heated to 50°C, and stirred with the propeller for 10 minutes, followed by immediate cooling to 22°C. The time required for heating to 50°C was 10 minutes, while the time required for cooling to 22°C was 5 minutes. After cooling, the crude slurry was adjusted to pH 4.8 with 35% hydrochloric acid and, after stirring at 22°C for additional 10 minutes, the separation-precipitation rate of the insoluble fraction was measured according to the same manner as in Example 1. Further, the ratio of 7S globulin and 11S globulin was determined according to the same manner as in Example 1 with respect to the soluble fraction obtained by centrifugation at 5,000 G for 5 minutes. On the other hand, water (7 fold by weight of defatted soybeans) was added to the insoluble fraction, and protein was extracted at 22°C by stirring with a homo-mixer for 30 minutes while the pH was maintained at 7.2 by adding a 20% aqueous sodium hydroxide solution, and the soluble fraction obtained by centrifugation at 5,000 G for 5 minutes was assayed by SDS-polyacrylamide electrophoresis to determine the ratio of 7S globulin and 11S globulin.

The ratios of 7S globulin and 11S globulin in the soluble fraction and insoluble fraction, respectively, are shown in Table 5, and the separation-precipitation rate of the insoluble fraction is shown in Table 6.

Comparative Example 3

The ionic strength of a defatted soybean slurry prepared according to the same manner as in Example 3 was adjusted to 0.17 with sodium chloride, and the slurry was stirred at 22°C for 30 minutes. Then, pH of the defatted soybean slurry was adjusted to 5.3 with 35% hydrochloric acid, followed by stirring at 22°C for 25 minutes. Then, pH of the slurry was adjusted to 4.8 with a 20% aqueous sodium hydroxide solution and, after stirring at 22°C for 10 minutes, the separation-precipitation rate of the insoluble fraction was measured, and the ratios of 7S globulin and 11S globulin in the soluble fraction and insoluble fraction were measured according to the same manner as Example 1.

The ratios of 7S globulin and 11S globulin in the soluble fraction and insoluble fraction are shown in Table 5, and the separation-precipitation rate of the insoluble fraction is shown in Table 6.

Table 5

Ratio between 7S globulin and 11S globulin (7S:11S)

| | Soluble fraction | Insoluble fraction |
|---|---|---|
| With heating | 92:8 | 9:91 |
| Without heating | 91:9 | 10:90 |

Table 6

Separation-precipitation rate of insoluble fraction

|  | With heating | Without heating |
|---|---|---|
| Separation-precipitation rate ($\mu$m/sec) | 395 | 84 |

As seen from the results in Example 3 and Comparative Example 3, it is clear that separation of the soluble fraction and the insoluble fraction is also facilitated by adjusting the heat treatment temperature and ionic strength under acidic conditions when the defatted soybean slurry is used.

Example 4

The ionic strength of a 5% curd slurry prepared according to the same manner as in Example 1 was adjusted to 0.17 with sodium chloride. After heating the slurry according to the same manner as in Example 3 (the slurry was stirred at 22°C for 30 minutes with a propeller without adjusting pH, pH was adjusted to 5.3, and the slurry was heated to 50°C, stirred with a propeller followed by immediate cooling to 22°C). Then, pH was adjusted to 5.4 with a 20% aqueous sodium hydroxide solution (pH for separation of the curd slurry should be higher than that for separation of the defatted soybean slurry for obtaining fractions with the same ratios of 7S globulin and 11S globulin from the defatted soybean slurry and curd slurry, when the ionic strength of the defatted soybean slurry was the same as that of the curd slurry) followed by stirring at 22°C for additional 10 minutes. The separation-precipitation rate of the insoluble fraction was measured according to the same manner as in Example 1, and the ratios of 7S globulin and 11S globulin in the soluble fraction and insoluble fraction were determined by SDS-polyacrylamide electrophoresis.

The ratios of 7S globulin and 11S globulin in the soluble fraction and insoluble fraction are shown in Table 7, and the separation-precipitation rate of the insoluble fraction is shown in Table 8.

Table 7

Ratio between 7S globulin and 11S globulin (7S:11S)

|  | Soluble fraction | Insoluble fraction |
|---|---|---|
| Curd slurry | 93:7 | 9:91 |

Table 8

Separation-precipitation rate of insoluble fraction

|  | Curd slurry |
|---|---|
| Separation-precipitation rate ($\mu$m/sec) | 156 |

As seen from the results in Example 3 and Example 4, it is clear that separation is much facilitated with a higher separation-precipitation rate of the insoluble fraction in case of using the defatted soybean slurry in the solution containing soybean protein.

Example 5

The ionic strength of a 5% curd slurry prepared by the same method as in Example 1 was adjusted to 0.14 with sodium chloride, and the slurry was stirred at 22°C for 30 minutes without adjusting the pH (about pH 4.5) followed by heating to 50°C. The time required for raising the temperature to 50°C was 10 minutes. After the temperature was raised to 50°C, pH of the slurry was adjusted to 5.3 with a 20% aqueous sodium hydroxide solution, and the slurry was heated for additional 20 minutes followed by cooling to 22°C. The time required for cooling was 5 minutes. After cooling, the slurry was centrifuged at 5,000 G for 10 minutes to obtain the soluble fraction and insoluble fraction.

The soluble fraction obtained was adjusted to pH 4.5 was 35% hydrochloric acid, and centrifuged at 3,000 G for 10 minutes to obtain a precipitated fraction. Then, to the precipitated fraction was added water, and the mixture was neutralized with a 20% aqueous sodium hydroxide solution and freeze-dried to obtain a 7S globulin-rich fraction.

On the other hand, 5 fold by weight of ion-exchange water was added to the insoluble fraction obtained by centrifugation at 5,000 G for 10 minutes, and the mixture was extracted by stirring at 22°C for 30 minutes with a propeller while pH of the mixture was maintained at 6.8 with a 20% aqueous sodium hydroxide solution. Then, the mixture was centrifuged at 5,000 G for 10 minutes, and pH of the supernatant obtained was adjusted to 4.5 with 35% hydrochloric acid, followed by centrifugation at 3,000 G for 10 minutes to obtain a precipitated fraction. Then, to the precipitated fraction was added water, and the mixture was neutralized with a 20% aqueous sodium hydroxide solution, and freeze-dried to obtain a 11S globulin-rich fraction.

The ratio of 7S : 11S of each fraction as determined by SDS-polyacrylamide electrophoresis, the purity of 7S globulin (SPE standard) in the 7S globulin-rich fraction and the purity of 11S globulin (SPE standard) in the 11S globulin-rich fraction, the amount of the chloroform-methanol oil content, the corrected purity of 7S globulin, the corrected purity of 11S globulin, and the content of phytic acid in each fraction are shown in Table 9. Table 10 shows the separation-precipitation rate of the insoluble fraction measured according to the same manner as in Example 1.

Table 9

Composition of each fraction (unit: %)

|  | Soluble fraction | Insoluble fraction |
|---|---|---|
| 7S:11S | 96:4 | 13:87 |
| Purity (SPE standard) | 93.0 | 82.9 |
| Crude protein (per dry weight) | 95.7 | 94.2 |
| Chloroform-methanol oil content (per dry weight) | 0.65 | 1.30 |
| Corrected purity | 87.0 | 72.1 |
| Phytic acid (per dry weight) | 2.1 | 2.0 |

Table 10

Separation-precipitation rate of insoluble fraction

|  | Without phytase treatment |
|---|---|
| Separation-precipitation rate ($\mu$m/sec) | 182 |

As seen from the results above, it is clear that the fractions of highly purified 7S and 11S globulins containing little amount of the chloroform-methanol fraction, or containing little amount of the oil-body-associated protein can be readily obtained by the process of the present invention.

From the results of Example 1 in which the sample solution was immediately cooled without holding the temperature after the heat treatment and from the results in Example 5 in which the temperature was maintained for 20 minutes after heat treatment, it is evident that maintaining the temperature after the heat treatment can improve the separation-precipitation rate.

Example 6

The ionic strength of a 5% curd slurry prepared according to the same manner as in Example 1 was adjusted to 0.14 with sodium chloride, the slurry was stirred at 22°C for 30 minutes without adjusting the pH (about pH 4.5) followed by heating to 50°C. The time required for heating was 10 minutes. When the temperature was raised to 50°C, pH of the slurry was adjusted to 5.3 with a 20% aqueous sodium hydroxide solution. Then, phytase (Sumizyme PHY, manufactured by Shin Nihon Chemical Co. Ltd.) was added in a proportion of 0.2% by weight based on the weight of defatted soybeans, and the slurry was stirred for additional 20 minutes with a propeller. Then, the slurry was cooled to 22°C. The time required for cooling was 5 minutes. After cooling, the slurry was centrifuged at 5,000 G for 10 minutes to obtain a soluble fraction and an insoluble fraction. The soluble fraction obtained was adjusted to pH 4.5 with 35% hydrochloric acid, followed by centrifugation at 3,000 G for 10 minutes to obtain a precipitated fraction. Then, to the precipitated fraction was added water, and the mixture was neutralized with a 20% aqueous sodium hydroxide solution, and freeze-dried to obtain a 7S globulin-rich fraction.

On the other hand, 5 fold by weight of ion-exchange water was added to the insoluble fraction obtained after centrifugation at 5,000 G for 10 minutes. The mixture was stirred at 22°C with a propeller, and was extracted for 30 minutes while maintaining pH at 6.8 with a 20% aqueous sodium hydroxide solution. Then, the mixture was centrifuged at 5,000 G for 10 minutes, the supernatant obtained was adjusted to pH 4.5 with 35% hydrochloric acid, followed by centrifugation at 3,000 G for 10 minutes to obtain a precipitated fraction. To the precipitated fraction was added water, and the mixture was neutralized with a 20% aqueous sodium hydroxide solution, and freeze-dried to obtain an 11S globulin-rich fraction.

The ratio of 7S : 11S of each fraction as determined by SDS-polyacrylamide electrophoresis, the purity of 7S globulin (SPE standard) in the 7S globulin-rich fraction and the purity of 11S globulin (SPE standard) in the 11S globulin-rich fraction, the amount of the chloroform-methanol oil content, the corrected purity of 7S globulin, the corrected purity of 11S globulin, and the content of phytic acid in each fraction obtained are shown in Table 11. Table 12 shows the separation-precipitation rate of the insoluble fraction measured according to the same manner as in Example 1.

Table 11

Composition of each fraction (unit: %)

|  | Soluble fraction | Insoluble fraction |
|---|---|---|
| 7S:11S | 97:3 | 14:86 |
| Purity (SPE standard) | 94.0 | 81.9 |
| Crude protein (per dry weight) | 96.2 | 92.4 |
| Chloroform-methanol oil content (per dry weight) | 0.61 | 1.24 |
| Corrected purity | 88.3 | 71.7 |
| Phytic acid (per dry weight) | 0.05 | 0.12 |

Table 12

Separation-precipitation rate of insoluble fraction

|  | With phytase treatment |
|---|---|
| Separation-precipitation rate ($\mu$m/sec) | 248 |

As seen from the results in Examples 5 and 6, it is clear that concomitant use of decomposition of phytic acid with phytase facilitates separation of the soluble fraction and the insoluble fraction and makes it possible to obtain the 7S globulin-rich fraction and the 11S globulin-rich fraction, both containing less phytic acid.

Industrial Applicability

As described hereinabove, according to the present invention, a 7S globulin-containing soluble fraction and a 11S globulin-containing insoluble fraction can be simply and readily fractionated in an industrial scale by combining adjustment of the ionic strength and heat treatment under acidic conditions of a solution containing soybean protein.

**Claims**

1. A process for producing soybean protein, which comprises heating a solution containing the soybean protein under acidic conditions, and then fractionating it into a soluble fraction and an insoluble fraction at an ionic strength of 0.02 or more and a pH of 4.5 or higher but lower than 5.6.

2. The process according to claim 1, wherein the solution containing the soybean protein is an aqueous slurry of defatted soybeans, defatted soybean milk obtained from the slurry, a slurry of acid-precipitated soybean protein, or a solution of soybean protein isolate.

3. The process according to claim 1, wherein the acidic conditions are those at pH 3.8 to 6.8.

4. The process according to claim 1, wherein the heating is performed at 30 to 75°C.

5. The process according to claim 1, which further comprises fractionating 7S globulin protein from the soluble fraction obtained by the fractionation in claim 1, wherein a ratio of 7S globulin/(11S globulin + 7S globulin) of said 7S globulin protein is 0.5 or more, and a content of a polar lipid extracted by a mixed solvent of chloroform and methanol (chloroform : methanol = 2 : 1) in the solid content of said 7S globulin protein is 1% by weight or less.

**6.** The process according to claim 1, which further comprises fractionating 11S globulin protein from the insoluble fraction obtained by the fractionation in claim 1, wherein a ratio of 11S globulin/(11S globulin + 7S globulin) of said 11S globulin protein is 0.7 or more, and a content of a polar lipid extracted with a mixed solvent of chloroform and methanol (chloroform : methanol = 2 : 1) in the solid content of said 11S globulin protein is 2% by weight or less.

**Patentansprüche**

**1.** Verfahren zur Herstellung von Sojabohnenprotein, welches das Erwärmen einer Lösung, die das Sojabohnenprotein unter sauren Bedingungen enthält, und dann Fraktionieren hiervon in eine lösliche Fraktion und eine unlösliche Fraktion bei einer Ionenstärke von 0,02 oder mehr und einem pH-Wert von 4,5 oder höher, jedoch niedriger als 5,6, umfaßt.

**2.** Verfahren gemäß Anspruch 1, worin die Lösung, die das Sojabohnenprotein enthält, eine wäßrige Aufschlämmung von entfetteten Sojabohnen, entfettete Sojabohnenmilch, erhalten aus der Aufschlämmung, eine Aufschlämmung von Säure-ausgefälltem Sojabohnenprotein oder eine Lösung eines Sojabohnenproteinisolats ist.

**3.** Verfahren gemäß Anspruch 1, worin die sauren Bedingungen diejenigen bei einem pH-Wert von 3,8 bis 6,8 sind.

**4.** Verfahren gemäß Anspruch 1, worin das Erwärmen bei 30 bis 75°C durchgeführt wird.

**5.** Verfahren gemäß Anspruch 1, welches ferner das Fraktionieren von 7S-Globulinprotein aus der löslichen Fraktion umfaßt, die aus der Fraktionierung in Anspruch 1 erhalten wird, worin das Verhältnis 7S-Globulin/(11S-Globulin + 7S-Globulin) des 7S-Globulinproteins 0,5 oder mehr beträgt und der Gehalt eines polaren Lipids, extrahiert mit einem gemischten Lösungsmittel aus Chloroform und Methanol (Chloroform:Methanol = 2:1), in dem Feststoffgehalt des 7S-Globulinproteins 1 Gew.% oder weniger beträgt.

**6.** Verfahren gemäß Anspruch 1, welches ferner das Fraktionieren von 11S-Globulinprotein aus der unlöslichen Fraktion umfaßt, die aus der Fraktionierung in Anspruch 1 erhalten wird, worin das Verhältnis 11S-Globulin/(11S-Globulin + 7S-Globulin) des 11S-Globulinproteins 0,7 oder mehr beträgt und der Gehalt eines polaren Lipids, extrahiert mit einem gemischten Lösungsmittel aus Chloroform und Methanol (Chloroform:Methanol = 2:1), in dem Feststoffgehalt des 11S-Globulinproteins 2 Gew.% oder weniger beträgt.

**Revendications**

**1.** Procédé pour la production d'une protéine de soja qui comprend le chauffage d'une solution contenant la protéine de soja dans des conditions acides et le fractionnement subséquent de celle-ci en une fraction soluble et en une fraction insoluble à une force ionique de 0,02 ou supérieure et à un pH de 4,5 ou supérieur mais inférieur à 5,6.

**2.** Procédé selon la revendication 1, dans lequel la solution contenant la protéine de soja est une suspension aqueuse de sojas dégraissés, du lait de soja écrémé obtenu à partir de la suspension, une suspension de protéine de soja acide-précipitée ou une solution de produit d'isolement de protéine de soja.

**3.** Procédé selon la revendication 1, dans lequel les conditions acides sont celles d'un pH de 3,8 à 6,8.

**4.** Procédé selon la revendication 1, dans lequel le chauffage est réalisé à de 30 à 75°C.

**5.** Procédé selon la revendication 1 qui comprend de plus le fractionnement de protéine de globuline 7S à partir de la fraction soluble obtenue par le fractionnement dans la revendication 1, dans lequel un rapport de globuline 7S/ (globuline 11S + globuline 7S) de ladite protéine de globuline 7S est de 0,5 ou supérieur et une teneur d'un lipide polaire extrait par un solvant mixte de chloroforme et de méthanol (chloroforme:méthanol = 2:1) dans le contenu solide de ladite protéine de globuline 7S est de 1 % en poids ou inférieure.

**6.** Procédé selon la revendication 1 qui comprend de plus le fractionnement de protéine de globuline 11S à partir de la fraction insoluble obtenue par le fractionnement dans la revendication 1, dans lequel un rapport de globuline 11S/ (globuline 11S + globuline 7S) de ladite protéine de globuline 11S est de 0,7 ou supérieur et une teneur d'un lipide polaire extrait avec un solvant mixte de chloroforme et de méthanol (chloroforme:méthanol = 2:1) dans le contenu solide de ladite protéine de globuline 11S est de 2 % en poids ou inférieure.

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 03063608 A
- US 4368151 A
- EP 1323353 A1
- JP 55124457 A
- JP 48056843 A
- JP 49031843 A
- JP 58036345 A
- JP 5043597 A
- JP 61187755 A
- US 6171640 B1
- WO 0228198 A1

**Non-patent literature cited in the description**

- *Breeding Science,* 1996, vol. 46, 11
- *Breeding Science,* 2000, vol. 50, 101
- *Biosci. Biotechnol. Biochem.,* 1998, vol. 62 (5), 935-940
- *Biosci. Biotechnol. Biochem.,* 1988, vol. 62 (5), 935-940
- **LAEMMLI.** *Nature,* 1970, vol. 227, 680
- *Cereal Chemistry,* 1986, vol. 63, 475-478